# EUROPEAN PATENT APPLICATION

(11) **EP 4 449 889 A1**
(43) Date of publication of application: **23.10.2024**
(21) Application number: 22907486.9
(22) Date of filing: 14.12.2022
(51) Int. Cl.: A23L 33/105, A61K 31/352, A61K 36/31, A61P 25/02, A61P 25/20

(54) **COMPOSITION FOR IMPROVING SLEEP QUALITY OR CONTROLLING AUTONOMIC NERVOUS SYSTEM**

(30) Priority: 15.12.2021 JP 2021203424
(71) Applicant: Otsuka Pharmaceutical Co., Ltd., Tokyo 101-8535 (JP)
(72) Inventor: OKADA, Shinpei, Tomi-shi, Nagano 389-0402 (JP); HANDA, Shuichi, Tomi-shi, Nagano 389-0402 (JP); SAITO, Bungo, Tomi-shi, Nagano 389-0402 (JP); KANNO, Teruyuki, Tomi-shi, Nagano 389-0402 (JP); IKEDA, Yasutaka, Osaka-shi, Osaka 540-0021 (JP); MIZOKAMI, Tsubasa, Osaka-shi, Osaka 540-0021 (JP)
(74) Representative: Hoffmann Eitle
(86) International application number: PCT/JP2022/046046
(87) International publication number: WO 2023/112963

(57) **Abstract**

A novel means for improving sleep quality etc. is provided. Provided is a composition for sleep quality improvement that contains kaempferol and/or a glycoside thereof.

## Description

### Technical Field

Disclosed are techniques for improving sleep quality and regulating the autonomic nervous system.

### Background Art

In recent years, many people feel that they do not get satisfactory sleep due to the stress of their social environment and personal life. Poor quality sleep is known to lead to reduced concentration, lowered immunity, and increased risk of suffering from mental diseases and lifestyle-related diseases. Measures to improve sleep quality are required.

To improve sleep, taking pharmaceutical sleeping pills or Chinese herbal medications as well as improvements in bedding and environment during sleeping have been proposed. Since sleep conditions are also largely influenced by dietary habits, attempts have also been made to develop foods that have sleep-improving effects (Patent Literature (PLT) 1 to 3).

The autonomic nervous system is divided into the sympathetic nervous system and the parasympathetic nervous system. Maintaining the balance between the sympathetic nervous system and the parasympathetic nervous system maintains mental and physical health. If a state of mental or physical stress persists for a long time, the sympathetic nervous system remains dominant and the parasympathetic nervous system gradually becomes dysfunctional, causing an imbalance of the autonomic nervous system and resulting in a state of impaired autonomic nervous system function. In a state of impaired autonomic nervous system function, mental and physical resilience is reduced. Not only physical symptoms, such as headache and malaise, but also mental symptoms, such as anxiety and depression, appear. The state of impaired autonomic nervous system function can also cause meteoropathy, by which the body cannot keep up with changes in the weather and environment, causing headaches and other physical disorders. To counteract these symptoms, it is believed that by making the parasympathetic activity dominant, the body and mind can be relaxed, stress can be reduced, and hyperactive mental states can be calmed; additionally, physical and mental fatigue can be recovered from, and meteoropathy can be alleviated (Patent Literature (PTL) 4).

### Citation List

### Patent Literature

PTL 1: JP2016-188234A
PTL 2: JP2005-281201A
PTL 3: WO2013/051727
PTL 4: JP2018-011730A

### Non-patent Literature

NPL 1: Jan Cosgrave et al., Revisiting nocturnal heart rate and heart rate variability in insomnia: A polysomnography-based comparison of young self-reported good and poor sleepers, Journal of Sleep Research, 18, 2021, 1-12

### Summary of Invention

### Technical Problem

An object of the present invention is to provide a new means for improving sleep quality. Another object of the present invention is to provide a new means for regulating the autonomic nervous system. A further object of the present invention is to provide a means for improving or preventing mental or physical disorders. Still another object of the present invention is to provide a means for alleviating, preventing, or treating meteoropathy.

### Solution to Problem

The present inventors conducted intensive research to achieve the above objects, and found that kaempferol (also referred to as "KMP") has the effects of improving sleep quality, lowering the sleeping heart rate, and regulating the autonomic nervous system, and is effective in alleviating or treating meteoropathy. As a result of further research and consideration based on this finding, the inventions represented below are provided.

Item 1
   A composition for sleep quality improvement, comprising kaempferol and/or a glycoside thereof.
Item 2
   A composition for lowering of sleeping heart rate, the composition comprising kaempferol and/or a glycoside thereof.
Item 3
   The composition according to Item 1, wherein the sleep quality improvement is achieved by lowering of sleeping heart rate.
Item 4
   A composition for regulating the autonomic nervous system, comprising kaempferol and/or a glycoside thereof.
Item 5
   A composition for alleviation, prevention, or treatment of meteoropathy, comprising kaempferol and/or a glycoside thereof.
Item 6
   The composition according to any one of Items 1 to 5, which is a food, beverage, drug, or quasi-drug.

### Advantageous Effects of Invention

In one embodiment, sleep quality can be improved. In another embodiment, the autonomic nervous system can be regulated. In a further embodiment, meteoropathy can be alleviated, prevented, or treated.

### Brief Description of Drawings

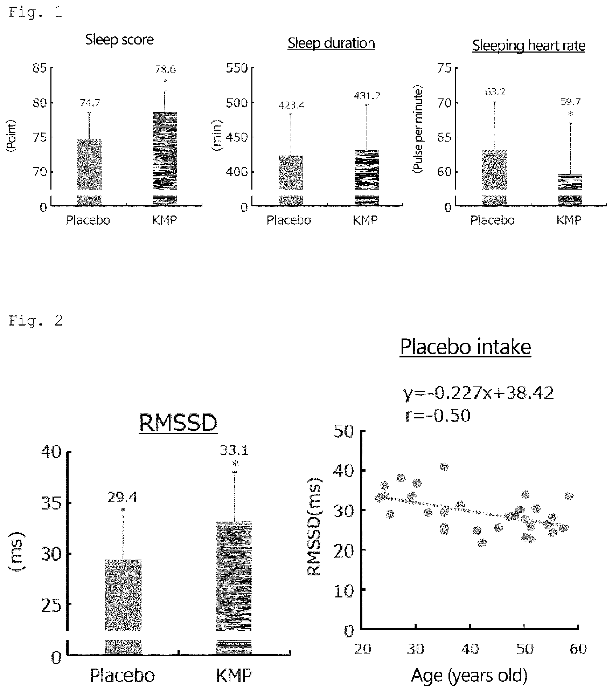

Fig. 1 shows measurements of the sleep score, sleep duration, and sleeping heart rate (the mean ± standard deviation) of subjects taking KMP and those taking a placebo. "*" means "P<0.05 vs placebo." The sleep duration and sleeping heart rate are the mean value per day.

The left graph in Fig. 2 shows measurements of the RMSSD (ms) (the mean ± standard deviation) of subjects taking KMP and those taking a placebo. "*" means "P<0.05 vs placebo." The right graph in Fig. 2 shows measurements of the RMSSD (ms) of the subjects taking a placebo on the ordinate, and the age of the subjects on the abscissa.

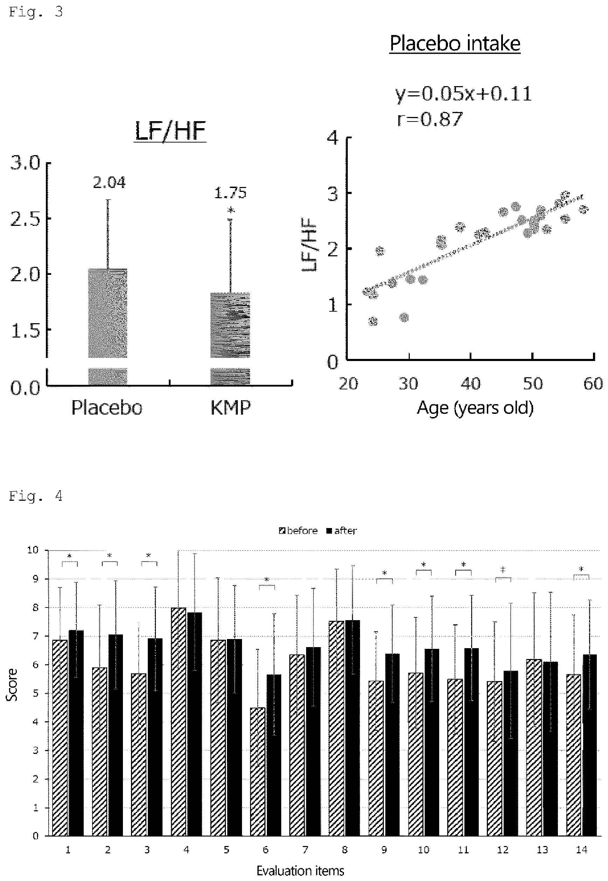

The left graph in Fig. 3 shows measurements of the LF/HF ratio (the mean ± standard deviation) of subjects taking KMP and those taking a placebo. "*" means "P<0.05 vs placebo." The right graph in Fig. 3 shows the LF/HF ratio of the subjects taking a placebo on the ordinate, and the age of the subjects on the abscissa.

Fig. 4 shows the results of evaluation made by all the subjects (159 subjects) before KMP intake and on day 30 from the start of KMP intake. The ordinate shows the score (the mean ± standard deviation). The abscissa shows the evaluation items (1. overall physical condition, 2. ease in sleep, 3. ease in waking up, 4. appetite, 5. abdominal condition/bowel movements, 6. overall fatigue, 7. mental stability, 8. motivation, 9. recovery from fatigue, 10. muscle condition, 11. breathing (shortness of breath), 12. practice amount, 13. practice intensity, 14. performance achievement. * means P <0.05; and ‡ means P <0.1.

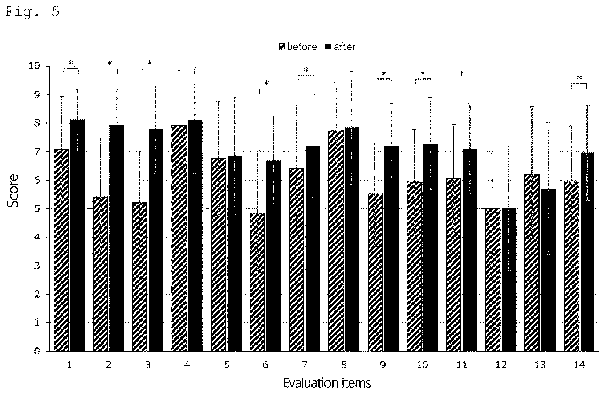

Fig. 5 shows the results of evaluation made by swimmers (50 persons) before KMP intake and on day 30 from the start of KMP intake. The ordinate shows the score (the mean ± standard deviation). The abscissa shows the evaluation items (1. overall physical condition, 2. ease in sleep, 3. ease in waking up, 4. appetite, 5. stomach/bowel movements, 6. overall fatigue, 7. mental stability, 8. motivation, 9. recovery from fatigue, 10. muscle condition, 11. breathing (shortness of breath), 12. practice amount, 13. practice intensity, 14. performance achievement). * means P <0.05; and ‡ means P <0.1.

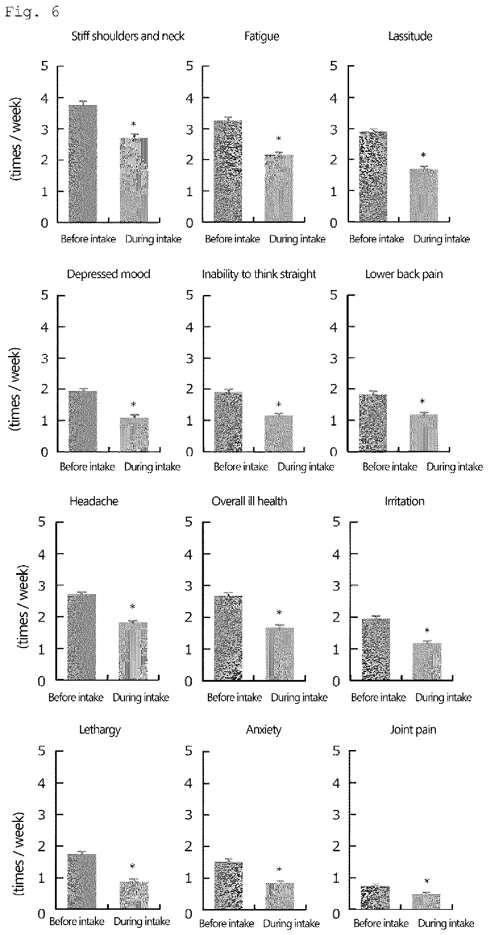

Fig. 6 shows measurement results of the frequency of each symptom of meteoropathy (stiff shoulders and neck fatigue, lassitude, headache, general ill health, irritation, depressed mood, inability to think straight, lower back pain, lethargy, anxiety, and joint pain) before KMP intake and during KMP intake (the mean ± standard error of all subjects). (*: P <0.01)

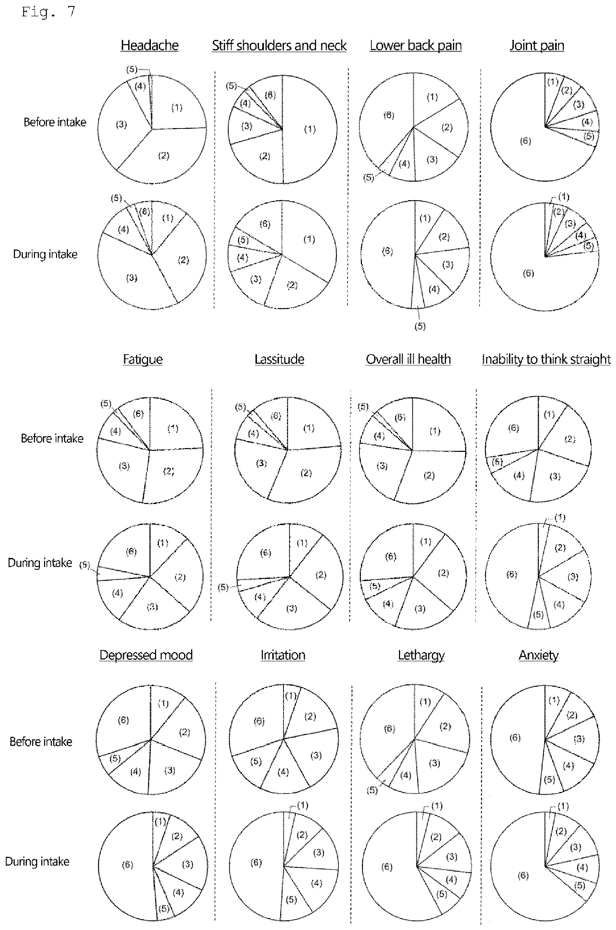

Fig. 7 shows the percentage of evaluations results regarding the duration of each symptom of meteoropathy (stiff shoulders and neck, fatigue, lassitude, headache, overall ill health, irritation, depressed mood, inability to think straight, lower back pain, lethargy, anxiety, and joint pain) before KMP intake and during KMP intake in all subjects, shown in pie charts. For each symptom, the top row shows the results before KMP intake, and the bottom row shows the results during KMP intake. The numerals shown in the pie charts have the following meanings: (1): almost all day; (2): about half a day; (3): several hours; (4): several tens of minutes, (5): several minutes, and (6): none.

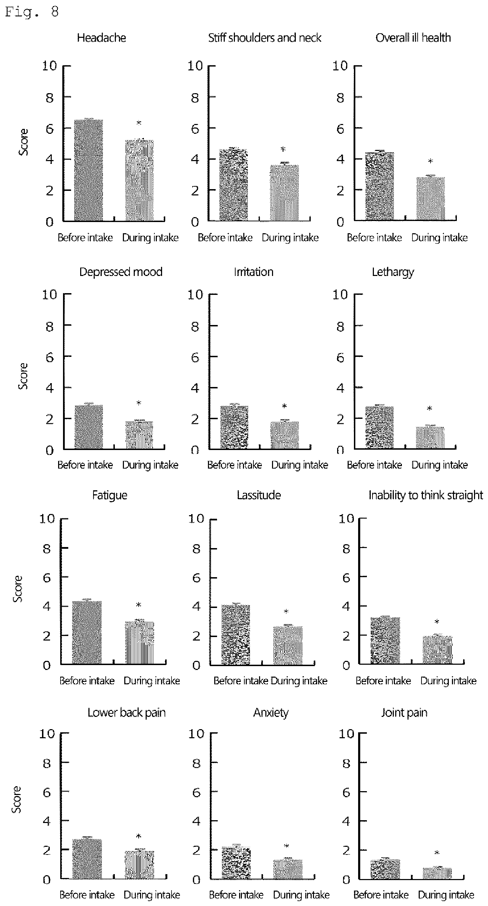

Fig. 8 shows measurement results of the degree of each symptom (stiff shoulders and neck, fatigue, lassitude, headache, overall ill health, irritation, depressed mood, inability to think straight, lower back pain, lethargy, anxiety, and joint pain) of meteoropathy before KMP intake and during KMP intake (the mean ± standard error of all subjects). (*: P <0.01)

### Description of Embodiments

Kaempferol is a substance represented by the structural formula shown below and is contained in many edible plants, such as tea, broccoli, grapefruit, cabbage, kale, beans, endive, leeks, tomatoes, strawberries, grapes, Brussels sprouts, apples, quinoa, and horseradish. Kaempferol may be isolated, or be present in an extract. In one embodiment, kaempferol is preferably derived from a plant selected from the group consisting of quinoa and horseradish.

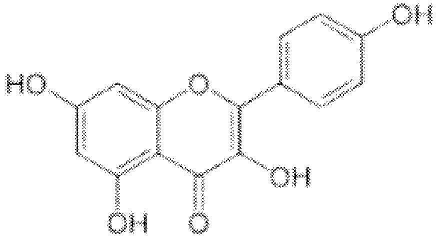

A glycoside of kaempferol means a compound in which a sugar chain having at least one (preferably one to three, more preferably one) sugar residue is glycosidically bonded to at least one (preferably one to two, more preferably one) hydroxy group. Preferred examples of sugar residues include a glucose residue, a mannose residue, a galactose residue, a fucose residue, a rhamnose residue, an arabinose residue, a xylose residue, a fructose residue, a glucuronic acid residue, and an apiose residue. The glycoside of kaempferol can be converted to its aglycone in vivo and thus can exhibit the same action as that of kaempferol.

The composition for sleep quality improvement, the composition for lowering of sleeping heart rate, the composition for regulating the autonomic nervous system, and/or the composition for alleviation, prevention, or treatment of meteoropathy preferably comprises kaempferol and/or a glycoside thereof as an active ingredient. In one embodiment, the composition preferably comprises kaempferol. The kaempferol and glycoside thereof can be obtained by extraction from plants according to known methods, or can also be commercially purchased and used.

In one embodiment, the composition for sleep quality improvement, the composition for lowering of sleeping heart rate, the composition for regulating the autonomic nervous system, and/or the composition for alleviation, prevention, or treatment of meteoropathy preferably does not contain dihydrokaempferol. The phrase "does not contain dihydrokaempferol" means that the content of dihydrokaempferol in the composition is below the detection limit.

In one embodiment, "sleep quality" can be measured by using the Pittsburgh Sleep Quality Index (PSQI). A lower PSQI score means that sleep quality is better, whereas a higher PSQI score means that sleep quality is worse. In one embodiment, kaempferol intake preferably reduces the PSQI score of subjects to lower than the standard cut-off value of '5.' In another embodiment, the PSQI score of subjects after kaempferol intake is preferably lower than that before kaempferol intake. It can be determined that sleep quality has improved based on a decrease in PSQI score.

In one embodiment, sleep quality improvement is preferably accompanied by lowering of sleeping heart rate. The sleeping heart rate refers to a heart rate during sleep, which also corresponds to a heart rate at rest. Lowering of the heart rate is considered to lead to recovery from fatigue, reduce the feeling of fatigue when waking up, and achieving good sleep. In one embodiment, as compared with the state without kaempferol intake, the sleeping heart rate (average value) with kaempferol intake is preferably lower by 1 bpm or more, 2 bpm or more, or 3 bpm or more. In one embodiment, as compared to the state without kaempferol intake, the sleeping heart rate with kaempferol intake is preferably lower by 30 bpm or less, 15 bpm or less, or 10 bpm or less. In one embodiment, "sleeping" or "during sleep" means a state in which the body is in a state of complete rest and has not moved substantially for approximately one hour.

The regulation of the autonomic nervous system by a composition for regulating the autonomic nervous system means increasing the activity of the parasympathetic nervous system relative to the activity of the sympathetic nervous system, eliminating the disturbances of the autonomic nervous system (mainly, a state of persistent sympathetic dominance), and making an appropriate balance between sympathetic nervous system activity and parasympathetic nervous system activity for mental and physical health. In one embodiment, the regulation of the autonomic nervous system means increasing the RMSSD(ms) value or reducing the LF/HF ratio. In one embodiment, the composition for regulating the autonomic nervous system can be used as an agent for autonomic nerve function improvement.

The composition for regulating the autonomic nervous system can be used to correct disturbances of the autonomic nervous system and thereby improve, alleviate, or prevent mental or physical disorders perceived by self to be related to climate (headache, dizziness, fatigue, joint pain, depressed mood, nausea, asthma attacks, stiff shoulders, numbness in the hands and feet, low blood pressure, lassitude (malaise), overall ill health, irritation, poor concentration (inability to think straight), lower back pain, lethargy, anxiety, etc.). In one embodiment, the composition for regulating the autonomic nerve system and/or the composition for alleviation, prevention, or treatment of meteoropathy can reduce the frequency of occurrence of, shorten the duration of, and/or alleviate the degree of, one or more symptoms of meteoropathy, and can be used for such a purpose.

The amount of kaempferol or a glycoside thereof contained in the composition for sleep quality improvement, the composition for lowering of sleeping heart rate, the composition for regulating the autonomic nervous system, and/or the composition for alleviation, prevention, or treatment of meteoropathy; the amount of kaempferol or a glycoside thereof to be taken per dose; and the amount of kaempferol or a glycoside thereof to be taken per day, can be suitably designed within the ranges in which the desired effect is achieved, and can be appropriately selected according to the form of the composition, frequency of administration, health condition of the subject, and the like. The administration period of the composition is not particularly limited as long as the desired effect is exhibited. The composition may be administered as a single dose or continuously. In one embodiment, the composition is desirably administered continuously over a certain period of time, for example, for 2 days, 3 days, 1 week, 10 days, 1 month, or 3 months or longer. The number of inoculations per day can be set to, for example, one, two, or three times.

In one embodiment, the composition for sleep quality improvement is preferably ingested by a subject in need of improving sleep quality. In one embodiment, the composition for lowering of sleeping heart rate is preferably ingested by a subject whose heart rate does not decrease or does not sufficiently decrease during sleep. In one embodiment, the composition for regulating the autonomic nervous system is preferably administered to a subject in need of autonomic nervous system regulation. Subjects in need of autonomic nerve regulation include, for example, subjects whose autonomic nervous system is disordered, subjects whose sympathetic nervous system activity is dominant over parasympathetic nervous system activity for a certain period of time, and subjects suffering from (or having the possibility of suffering from) meteoropathy. In one embodiment, the composition for alleviation, prevention, or treatment of meteoropathy is preferably ingested by a subject in need of alleviation, prevention, or treatment of meteoropathy. Examples of subjects in need of alleviation, prevention, or treatment of meteoropathy include persons suffering from physical or mental disorders perceived by the persons themselves to be related to the weather.

The amount of the kaempferol or glycoside thereof contained in the composition for sleep quality improvement, the composition for lowering of sleeping heart rate, the composition for regulating the autonomic nervous system, and/or the composition for alleviation, prevention, or treatment of meteoropathy is, for example, 0.1 mg to 200 mg, preferably 0.5 mg to 100 mg, more preferably 1 mg to 30 mg, and most preferably 2 mg to 15 mg, as a value in terms of kaempferol, although it varies depending on the total weight of the composition. The lower limit of the value in terms of kaempferol is, for example, 0.1 mg, 0.5 mg, 1 mg, 2 mg, or 2.5 mg, and the upper limit of the value in terms of kaempferol is, for example, 200 mg, 150 mg, 100 mg, 50 mg, 30 mg, 25 mg, or 15 mg. These upper limits and lower limits can be combined in any way.

The term "value in terms of kaempferol" means a value obtained by converting the amount of the glycoside of kaempferol into the amount of kaempferol as its aglycone. Specifically, the value in terms of kaempferol can be obtained by multiplying the number of moles of the glycoside, which is obtained by dividing the amount of the glycoside by its molecular weight, by the molecular weight of the aglycone.

The intake amount of the composition for sleep quality improvement, the composition for lowering of sleeping heart rate, the composition for regulating the autonomic nervous system, and/or the composition for alleviation, prevention, or treatment of meteoropathy can be individually set, based on the sleep conditions, age, sex, body weight, diet, and like conditions of the human who ingests the composition. The daily intake of the composition can be, for example, 0.1 mg to 600 mg, preferably 0.5 mg to 200 mg, and more preferably 1 mg to 100 mg, as a value in terms of kaempferol. The lower limit of the value in terms of kaempferol is, for example, 0.1 mg, 0.5 mg, 1 mg, 2 mg, or 2.5 mg. The upper limit of the value in terms of kaempferol is, for example, 600 mg, 300 mg, 200 mg, 150 mg, 100 mg, 50 mg, 30 mg, 25 mg, 15 mg, 10 mg, 5 mg, 3 mg, or 2.5 mg. These upper limits and lower limits can be combined in any way. The frequency of intake of the composition may be once or multiple times (e.g., 2, 3, 4, or 5 times) a day.

The composition for sleep quality improvement, the composition for lowering of sleeping heart rate, the composition for regulating the autonomic nervous system, and/or the composition for alleviation, prevention, or treatment of meteoropathy can be a food or beverage, a pharmaceutical composition, or a quasi-drug. The food or beverage includes foods with function claims, foods for specified health uses, health foods, dietary supplements (supplements), foods for medical purposes, foods for the sick, and the like. The composition for sleep quality improvement, the composition for lowering of sleeping heart rate, the composition for regulating the autonomic nervous system, and/or the composition for alleviation, prevention, or treatment of meteoropathy can be produced based on a known formulation technique and a known food manufacturing technique.

The dosage form of the composition for sleep quality improvement, the composition for lowering of sleeping heart rate, the composition for regulating the autonomic nervous system, and/or the composition for alleviation, prevention, or treatment of meteoropathy is not particularly limited and can be appropriately designed according to the purpose. The composition can be, for example, in the form of tablets, granules, capsules, powders, chewable tablets, confectionery (e.g., cookies, biscuits, chocolate confections, chips, cake, gum, candies, gummy candies, buns, adzuki-bean jelly, pudding, jelly, yogurt, ice cream, and sherbet), bread, noodles, cooked rice, cereal foods, drinks (e.g., liquids, soft drinks, carbonated drinks, nutritional drinks, powdered drinks, fruit drinks, dairy drinks, and jelly drinks), soups (powder, freeze-dry), miso soups (powder, freeze-dry), or other common forms of food products.

The composition may consist only of kaempferol and/or a glycoside thereof, or can contain, in addition to the kaempferol and/or glycoside thereof, any pharmaceutically acceptable carriers (e.g., excipients, binders, lubricants, stabilizers, diluents, bulking agents, thickeners, gelling agents, emulsifiers, coloring agents, flavorings, sweeteners, and additives).

In one embodiment, the subject can be a human. In one embodiment, the human can be 15 years old or older, 20 years old or older, 30 years old or older, 40 years old or older, 50 years old or older, 60 years old or older, 70 years old or older, or 80 years old or older.

In one embodiment, the composition for sleep quality improvement, the composition for lowering of sleeping heart rate, the composition for regulating the autonomic nervous system, and/or the composition for alleviation, prevention, or treatment of meteoropathy may be a composition whose product body, package, instruction manual, advertising materials, or items similar thereto have a statement thereon such as "has a sleep quality improvement effect," "reduces the heart rate during sleep," "regulates the autonomic nervous system," "alleviates, prevents, or treats meteoropathy or individual main symptoms associated with the meteoropathy," or other indications that the composition is useful for these symptoms.

In one embodiment, the composition containing kaempferol and/or a glycoside thereof can be used for any of the following other purposes/applications.
(a) Reducing menstrual pain.
(b) Improving or preventing menstrual cycle disturbances.
(c) Reducing or preventing abdominal tension during menstruation.
(d) Reducing premenstrual swelling (e.g., swelling of the legs).
(e) Improving or preventing depressed mood.
(f) Maintenance of mental stability.
(g) Alleviation or suppression of fatigue accumulation.
(h) Improved or sustained concentration.
(i) Alleviation or improvement of jet lag.
(j) Improved bowel movements.
(k) Improved immunity.
(l) Accelerated healing of injuries.
(m) Reduced body weight gain.

The dosage form of the composition and frequency of use (administration) when the composition is used for the above other purposes/applications can be the same as those for the composition for sleep quality improvement.

### Examples

The present invention is described below in more detail with reference to Examples. However, the present invention is not limited to these Examples.

### Test 1

A randomized, double-blind, placebo-controlled, crossover comparative test as summarized in the table below was conducted to examine the relationship between kaempferol, sleep, and the autonomic nervous system.

**Table 1**

| | |
|---|---|
| Subjects | 34 healthy adult men and women (17 women, 17 men) |
| Test samples | - Granular food not containing KMP (placebo) |
| | - Granular food containing 10 mg of KMP per pouch |
| Method of intake | Intake once a day, after breakfast, for two consecutive weeks. |
| Measurement items | - Sleep score, |
| | - Sleep duration, |
| | - REM sleep duration, |
| | - Deep sleep duration, |
| | - Sleeping heart rate, |
| | - RMSSD |
| | - LF/HF ratio |
| Measurement method | Subjects wore a "Fitbit Charge 4" wristwatch fitness tracker to measure each of the above items every day for two weeks. |

The granular foods used in the test were prepared according to the following procedure. Horseradish leaves were extracted twice with 50% aqueous ethanol at 50°C and concentrated under reduced pressure. The concentrate was purified with resin to obtain a solution. Hemicellulase "Amano" 90 and Lactase F "Amano" (produced by Amano Enzyme, Inc.) were added to the solution to allow the enzyme to act thereon while stirring at 50°C. The resulting reaction liquid was then heated to 80°C and maintained for 20 minutes to inactivate the enzyme. The resulting treated liquid was then freeze-dried to give an ethanol extract powder. This powder was combined with an equal amount (on a weight basis) of starch and erythritol and mixed into granules. The granules were packed in aluminum pouches in an amount of 116 mg (containing 10 mg of kaempferol) per pouch. The subjects ingested the contents of one pouch at one time (10 mg of kaempferol in total). The food used as a placebo was granules of starch and erythritol.

Fig. 1 shows measurement results of the sleep score, sleep duration, and sleeping heart rate. The results in Fig. 1 show that KMP intake significantly improves the sleep score and significantly reduces the sleeping heart rate, while no significant change in sleep duration occurs. The sleep score is an index of sleep quality on a scale of 0 to 100, with a score of 90 to 100 indicating very good sleep quality, a score of 80 to 89 indicating good sleep quality, a score of 60 to 79 indicating slightly low sleep quality, and a score of 60 or less indicating low sleep quality. The sleeping heart rate is known to correlate with sleep quality. Specifically, it has been reported that the higher the heart rate during sleep, the less satisfactory the sleep obtained is (i.e., the quality of sleep is low) (see Non-Patent Literature (NPL) 1). Fig. 1 shows that KMP intake reduces the heart rate by 3.5 bpm on average. This is generally consistent with the difference between the average sleeping heart rate in people with good quality sleep and that in people with poor sleep quality (Fig. 3 in NPL 1). These results indicate that KMP intake reduces the sleeping heart rate and improves sleep quality.

Fig. 2 shows measurement results of the RMSSD during sleep. RMSSD is the square root of the mean squared differences of successive adjacent R-R intervals (interval from one QRS wave to the next QRS wave) and is an index of vagal tone intensity. A rise in RMSSD indicates increased parasympathetic activity or decreased sympathetic activity. The results shown in Fig. 2 (left) shows that KMP intake leads to an increase in RMSSD value (that is, increased parasympathetic activity or decreased sympathetic activity). Fig. 2 (right) shows a tendency of RMSSD reduction with age (probably due to reduced parasympathetic function). This result is consistent with the tendency reported in Zhao H. et al., Ann Noninvasive Electrocardiol., 2015; 20 (2): 158-166. According to the formula shown in Fig. 2 (right), KMP intake is considered to rejuvenate the age of autonomic nervous system function by 16.3 years.

Fig. 3 shows measurement results of the LF/HF ratio during sleep. The LF/HF ratio is a ratio of low frequency (LF) to high frequency (HF) power and represents the overall balance between the sympathetic nervous system and the parasympathetic nervous system. A higher LF/HF ratio indicates a state of sympathetic nervous system dominance, whereas a lower LF/HF ratio indicates parasympathetic nervous system dominance. The results in Fig. 3 (left) show that KMP intake reduces the LF/HF ratio (i.e., leads to increased parasympathetic activity or decreased sympathetic activity). Fig. 3 (right) shows a tendency for the LF/HF ratio to increase with age (probably due to reduced parasympathetic nerve function), which is consistent with the tendency reported in Yeragani V. K. et al., Cardiovascular Research, 1997; 35 (1): 335-42. According to the formula shown in Fig. 3 (right), KMP intake is considered to rejuvenate the age of autonomic nervous system function by 5.8 years.

### Test 2

The following tests were carried out to investigate the functions of kaempferol.

**Table 2**

| | |
|---|---|
| Subject | 159 athletes, |
| | - 105 men and 54 women |
| | - 18 to 22 years old: 80 persons, 23 to 27 years old: 53 persons, 28 years old or order: 26 persons, |
| | - track-and-field athletes: 77 persons, Swimmers: 50 persons, other sportspeople: 32 persons (other |
| | sports include rugby, basketball, football, cycling, cross-country skiing, and alpine skiing) |
| Test sample | Granular food containing 10 mg of KMP per pouch (the same as that used in Test 1) |
| Method of intake | Intake once a day for 30 consecutive days |
| Evaluation items | - Overall physical condition, |
| | - Ease in sleep, |
| | - Ease in waking up, |
| | - Appetite, |
| | - Stomach/bowel movements, |
| | - Overall fatigue, |
| | - Mental stability, |
| | - Motivation, |
| | - Recovery from fatigue, |
| | - Muscle condition, |
| | - Breathing (shortness of breath), |
| | - Practice volume, |
| | - Practice intensity, |
| | - Performance achievement |
| Evaluation method | A questionnaire (on a scale of 0 to 10) was administered to each subject before and on day 30 after the start of intake of the test sample for each evaluation item using the following evaluation criteria. |

**Table 3**

| Evaluation criteria | |
|---|---|
| 1. Overall physical condition | 0 points: "very poor" to 10 points: "excellent" |
| 2. Ease in sleep | 0 points: "very poor" to 10 points: "excellent" |
| 3. Ease in waking up | 0 points: "very poor" to 10 points: "excellent" |
| 4. Appetite | 0 points: "very little" to 10 points: "sufficient" |
| 5. Belly/bowel movements | 0 points: "very poor" to 10 points: "excellent" |
| 6. Overall fatigue | 0 points: "very strong" to 10 points: "not at all" |
| 7. Mental stability | 0 points: "very depressed" to 10 points: "very confident" |
| 8. Motivation | 0 points: "not at all" to 10 points: "a great deal" |
| 9. Fatigue recovery | 0 points: "very slow" to 10 points: "very fast" |
| 10. Muscle condition | 0 points: "very poor" to 10 points: "excellent" |
| 11. Respiration (shortness of breath) | 0 points: "very hard" to 10 points: "very easy" |
| 12. Practice amount | 0 points: "less than planned" to 10 points: "more than planned" (practiced more than planned.) |
| 13. Practice intensity | 0 points: "weaker than planned" to 10 points: "stronger than planned" (pushed self.) |
| 14. Performance achievement | 0 points: "worse than ever" to 10 points: "better than ever" |

Fig. 4 shows the average of the evaluation results of all the subjects. Fig. 5 shows the results of the swimmers only. These results confirmed that there is a significant improvement in terms of ease in sleep and ease in waking up; and that interestingly, this improvement is particularly prominent in the swimmers. This result is considered to demonstrate that KMP intake improves sleep quality. The results also confirmed that KMP intake is useful in fatigue alleviation, recovery from fatigue, improvement of endurance (improved shortness of breath) and achievement of exercise performance.

In addition to the above score evaluations, an optional evaluation of the mental and physical state after intake of the test sample as compared to that before the intake was obtained in the form of comments. Typical comments are shown in the table below.

**Table 4**

| Subject No. | Comments relating to menstruation |
|---|---|
| 1 | Menstruation, which stopped for about six months, has come naturally. |
| 2 | Menstrual cycle, which tended to be delayed, has returned to normal. |
| 3 | Was amenorrheic, but menstrual cycle has become stable. |
| 4 | Menstrual pain has been relieved. Menstrual cycle irregularities has been improved. |
| 5 | Menstrual pain has been relieved. |
| 6 | Menstrual pain has been relieved and abdominal distension during menstruation has also been relieved. |
| 7 | Swollen feet before menstruation has been relieved. |

**Table 5**

| Subject No. | Comments relating to mental state |
|---|---|
| 8 | Feel better during practice. |
| 9 | Have become able to practice with a positive attitude and confidence. |
| 10 | Have become able to run more calmly than usual. |
| 11 | Feel more comfortable during running. |
| 12 | Feel more relaxed both during and outside of practice. |
| 13 | Feel more relaxed and confident, which leads to better running. |
| 14 | Have more motivation to practice. |
| 15 | Feel more highly motivated before a competition. |
| 16 | Feel more positive. |
| 17 | More mentally stable. |
| 18 | No longer feel depressed. |
| 19 | Feel less tired and more motivated to practice. |

**Table 6**

| Subject No. | Other comments |
|---|---|
| 20 | Feel less sleepy during the day, in particular, after morning practice. |
| 21 | Concentration during desk work after practice has improved. |
| 22 | Have improved concentration. |
| 23 | Symptoms of jet lag were alleviated. |
| 24 | The time taken to get rid of jet lag was reduced. |
| 25 | Used to get sick every year at the change of season, but this has disappeared. |
| 26 | The symptoms of seasonal colds were not seen. |
| 27 | Stools have improved. |
| 28 | Bowel movements have improved. |
| 29 | Bowel movements have improved. |
| 30 | Bowel movements have improved. |
| 31 | Healing from injuries is faster. |
| 32 | Healing of fractures is much faster. |
| 33 | Healing of wounds after tooth extraction is faster. |
| 34 | Easier body weight loss. |
| 35 | Easier to control body weight gain. |

As described above, KMP intake is expected to enjoy various functions, such as relief of menstrual pain, reduction of irregular menstrual cycle, reduction of premenstrual swelling, improvement of mental stability, suppression of depressed mood, increase of motivation, reduction of sleepiness, increase of concentration, reduction of jetlag, improvement of bowel movements, promotion of healing of injuries, and suppression of body weight gain.

### Test 3

The test shown below was conducted to investigate the functions of kaempferol associated with meteoropathy.

**Table 7**

| | |
|---|---|
| Subjects | 393 persons with meteoropathy (mental and physical disorders perceived by the persons themselves to be related to the weather) (349 women and 44 men) |
| Test sample | Granular food containing 10 mg of KMP per pouch (the same as that used in Test 1) |
| Method of intake | Intake once a day, after breakfast, for 4 consecutive weeks |
| Evaluation items | Frequency of occurrence, degree, duration, degree of improvement for each of the following symptoms: |
| | (a) Stiff shoulders |
| | (b) Fatigue |
| | (c) Lassitude |
| | (d) Headache |
| | (e) Overall ill health |
| | (f) Irritation |
| | (g) Depressed mood |
| | (h) Inability to think straight |
| | (i) Lower back pain |
| | (j) Lethargy |
| | (k) Anxiety |
| | (l) Joint pain |
| Evaluation method | Measured by administering a questionnaire before and after the start of intake of the test sample. The degree of each symptom was measured using a 0-10 point scale, with 0 being "not at all painful" and with 10 being "so painful that it interferes with daily life," with the subject responding to each item. |

Fig. 6 shows evaluation results of the frequency of occurrence of each evaluation item by all subjects. These results confirmed that the intake of KMP significantly reduces the frequency of all the following symptoms as compared to before: stiff shoulders and neck, fatigue, lassitude, headache, overall ill health, irritation, depressed mood, inability to think straight, lower back pain, lethargy, anxiety, and joint pain.

Fig. 7 shows the percentage of evaluation results regarding the duration of each symptom of meteoropathy by all subjects, shown in a pie chart. In Fig. 7, the upper row shows the evaluation results before KMP intake and the lower row shows the evaluation results during KMP intake. For all the symptoms, there was observed a tendency towards a significantly reduced duration of the symptom and an increased proportion of no occurrence of the symptom during KMP intake, as compared to those before KMP intake.

Fig. 8 shows evaluation results of the degree of each symptom of meteoropathy by all subjects. The results confirmed that as compared to the state before KMP intake, the intake of KMP significantly alleviated the degree of all of the following symptoms: stiff shoulders and neck, fatigue, lassitude, headache, overall ill health, irritation, depressed mood, inability to think straight, lower back pain, lethargy, anxiety, and joint pain, as compared to those before the intake.

In this way, the intake of KMP was confirmed to be effective in the treatment and improvement of meteoropathy; and KMP intake is considered to also be effective in the prevention of meteoropathy.

## Claims

1. A composition for sleep quality improvement, comprising kaempferol and/or a glycoside thereof.

2. A composition for lowering of sleeping heart rate, comprising kaempferol and/or a glycoside thereof.

3. The composition according to claim 1, wherein the sleep quality improvement is achieved by lowering of sleeping heart rate.

4. A composition for regulating the autonomic nervous system, comprising kaempferol and/or a glycoside thereof.

5. A composition for alleviation, prevention, or treatment of meteoropathy, comprising kaempferol and/or a glycoside thereof.

6. The composition according to any one of claims 1 to 5, which is a food, beverage, drug, or quasi-drug.
